# EUROPEAN PATENT SPECIFICATION

(11) **EP 0 267 539 B1**
(45) Date of publication and mention of the grant of the patent: **07.04.1993**
(21) Application number: 87116315.0
(22) Date of filing: 05.11.1987
(51) Int. Cl.: A61B 17/22

(54) **Transluminal microdissection device**
Einrichtung zum transluminalen Mikrosezieren
Dispositif d'angiorésection

(30) Priority: 12.11.1986 US 929956
(43) Date of publication of application: 18.05.1988
(73) Proprietor: Heart Technology, Inc., Bellevue, Washington 98005 (US)
(72) Inventor: Auth, David Christopher, Redmond Washington (US)
(74) Representative: VOSSIUS & PARTNER

(56) References cited:
- EP-A- 0 086 048
- EP-A- 0 177 782
- EP-A- 0 229 620
- DE-A- 2 933 266
- US-A- 3 809 093

## Description

The present invention relates to a mechanical device which is used in medical applications and which is capable of differentially cutting abnormal deposits from within a patient's vessels.

U.S. Patent No. 4,445,509 entitled METHOD AND APPARATUS FOR REMOVAL OF ENCLOSED ABNORMAL DEPOSITS which issued to David C. Auth on May 1, 1984 describes a rotary mechanical system for differentially cutting relatively hard intravascular deposits while sparing relatively soft, normal tissue. In the device described in that patent, a hollow channel was used for suction removal of debris generated during the cutting process in order to prevent the debris from acting as the nucleus for thrombogenesis or from occluding smaller vascular channels and thereby inhibiting the normal flow of life sustaining blood.

Suctioning of debris may not recover all of the cutting products if vascular flow is present in the artery being treated, since fluid motion at the cutting tip will immediately carry some debris downstream. U.S. Patent No. 4,207,874 entitled LASER TUNNELLING DEVICE which issued to D.S. Choy on June 17, 1980 describes an apparatus which removes intravascular deposits by using a laser to vaporize intravascular obstructions. When laser energy is used to vaporize debris, the laser may provide sufficent energy to release each constituent molecule from the host lattice or it may produce gaseous products within the solid matrix, thereby causing a rupture of the matrix and the release of smaller constituent particles of the mass. In the former case, the amount of energy required to uncouple each individual molecule is relatively large due to the binding energy of each molecule and to the large number of molecules per unit volume of obstructing mass. In the latter case, the released particles can be relatively large and may be capable of obstructing smaller vascular branches distal to the site of the treated obstruction.

In U.S. Patent No, 4,445,509, referred to above, the preferential cutting of hard deposits vis-a-vis soft normal tissue is a desirable feature. Unfortunately, harmful obstructing deposits can, on occasion, be soft. Frequently, such soft occluding deposits are also lacking in physical toughness, i.e., they lack the ability to recover after deformation. Muscular tissue tends to be rather tough and to be able to recover after significant elastic deformation. Thus, an additional physical property which may be considered for differentiating the cutting efficacy of a particular device is its ability to distinguish between soft (compliant), tough tissue, which will not break up as a result of local deformation, and soft, weak tissue, which will break up under local deformation. As taught in U.S. Patent No. 4,445,509, the differential cutting action derives from the ability of soft tissue to "dive" out of the way before it is caught in front of the cutting edge and cleaved off. The process of "diving" implies deformation which can decimate soft, weak tissue without seriously damaging soft, tough tissue. However, even soft, tough tissue can be cleaved if the rate at which the deformation required to escape cleavage exceeds the speed with which the tissue can move given its own inertia. Thus, increasing the surface speed of the cutting edge can eventually result in the ability to cleave soft, tough tissue. This distinction can be useful when it is desirable to cleave obstructive tissue masses which are soft and weak or soft and tough. Depending upon the local vessel anatomy, some damage to normal vessel endothelium or media may occur, and although less than desirable, that may well be a price worth paying to relieve the underlying obstructive condition. Since damage to endothelium and media occurs routinely in surgical vessel grafts which subsequently re-endothelize, the prognosis for rehealing of intima and media damaged adjacent to removed pathological material is good. Administration of drugs which suppress normal clotting may be required to inhibit thrombosis at the damage site during and after treatment.

When intravascular obstructions have a fibrous structure, there is a tendency to turn up a "scab" of material at the base of the cutting zone. Such scabs grow in size with additional cutting rather than being clipped off. They can present a problem if left within the artery, as they may flop across the arterial vessel and obstruct flow or they may become a nucleating site for thrombogenesis or regrowth of atheroma.

EP-A-229 620, which was published after the priority of the present application, describes a transluminal microdissection device similar to the present invention. It comprises an abrasive tipped rotating cutting tool for use in removing abnormal deposits within a patient's vessels, having a tip which is covered with a material such as diamond grit, and rotated at high speed to pulverize any abnormal deposits contained within the vessel. Figure 2 shows an exploded view of a tip 12 covered with an abrasive cutting material 14 which is used in the device disclosed in EP-A-229 620. The tip 12 is connected via a hollow flexible drive shaft 16 to a prime mover (not shown). This device further comprises a blunt tip 32 at the distal end of a guide rail 30 being connected to the tip of 12 and having a preformable portion 34.

The present invention provides a transluminal microdissection device comprising a rotatable, flexible drive shaft having a substantially ellipsoidal cutting burr attached thereto, said burr having a diameter which is greater than that of the drive shaft, an abrasive surface on said burr, said abrasive surface being comprised of an abrasive material attached only to the distal surface of said burr, and a rotatable prime mover which is capable of rotating said drive shaft at a rotation rate of from about 20,000 revolutions per minute to about 160,000 revolutions per minute, whereby particles small enough to pass through the capillaries of a person can be microdissected.

It has been found that tiny cutting surfaces which act as shovels operating at surface speeds of about 12 m/s (40 feet per second (ft/sec)) can snip off microscopic divots before a scab can grow to appreciable size. These tiny shovels are preferrably comprised of fragments of diamond crystal or grit. Other sharp grit could be used, but diamond is inexpensive in this format and provides good wear characteristics. When these crystalline fragments (shovels) are very small in size, they necessarily generate very small debris fragments. If the debris tissue fragments are sufficiently small in size, they will propagate through the tiniest vascular channels (capillary beds) without clogging them. Thus, when using 30 µm (micron) size diamond fragments, the chip size of the fragments can easily be less than 5 µm (microns), i.e., less than the size of a red blood cell, which, of course, propagates through the capillary network. A 5 µm (micron) size debris fragment contains many millions of constituent molecules. Accordingly, the energy required to produce such fragments is orders of magnitude less than would be required if a laser using molecular evaporation was employed. Releasing many calories of energy within a blood vessel (using a laser) carries an attendant high risk of vessel wall damage by thermal conduction and subsequent thermal necrosis.

In accordance with the present invention, an elliposoidal cutting head, or burr, similar in shape to that depicted in U.S. Patent No. 4,445,509 is coated with tiny diamond chips (shovels). The cutting head is rotated at a speed which, in conjunction with its geometrical' circumference, provides a surface velocity of approximately 12 m/s (40 ft/sec). It has been found that a burr of this type, operated at such a burr velocity, is able to cut soft material at a high removal rate, while generating microscopic particles (on the order of 5 microns or less) and leaving behind a tissue base having a smooth appearance. Such burrs can now be fabricated in sizes ranging from about .5 mm in diameter up to 6 mm diameter. To achieve a surface speed of 12 m/s (40 ft/sec) with a tip of 1.5 mm diameter requires a rotation rate of approximately 155,000 revolutions per minute (rpm).

Transmission of such high rates of rotation through a flexible catheter has recently been shown to be possible using 0,5 mm (.020") trifilar helically wound drive shaft spinning within a thin plastic tube using a solid steel shaft having a 0.23 mm (.009ʺ) diameter, which tapers down to less than 0.13 mm (.005ʺ) at the tip, as a stationary core or rail. Infusion of biocompatible saline through the plastic sheath provides cooling of the sliding interface during operation.

Using the same mechanical configuration but operating at reduced rpm allows the same device to preferentially cut hard material while sparing soft material. Operation at high rotation speed will, of course, cut hard material very well. Indeed, hard material is usually removed more easily at all speeds relative to soft material. The point is that at very high surface speeds (approximately 12m/sec (40 ft/sec) and above) even the soft tissue can be cut, whereas at lower speeds it is very difficult to remove, but the hard material can still be dissected. Thus, a single device whose speed is modulated becomes a multipurpose device capable of differential cutting or soft tissue cutting. This device has now been shown to work in a variety of an imal tissues varying from soft to hard while being flexibly conveyed through a plastic catheter.

In a preferred embodiment of the invention, the burr is coated with coarse abrasive material at its distal end and with finer abrasive material at distances more remote from the distal end, such that the coarse material acts to quickly abrade obstructions as the burr is advanced, the finer abrasive material being adapted more toward polishing the inner surfaces of the vessel. It is preferable that in the region of the burr having the widest diameter there be essentially no abrasive material in order to prevent a rotating burr which is not being advanced through a vessel from abrading through the sides of the vessel.

In the Drawing:
FIG. 1 depicts a preferred embodiment of the present invention;
FIG. 2 is an exploded side view of a tip of a similar device disclosed in EP-A-229 620;
FIG. 3 is a side view of a version of the embodiment of the invention which includes a variable grit angioplasty burr;
FIG. 4 is a side view of the embodiment of FIG. 3 in which the atraumatic tip of FIG. 3 is replaced by a preformable guide wire; and
FIG. 5 is a cross-sectional view of an embodiment including radial water spouts.

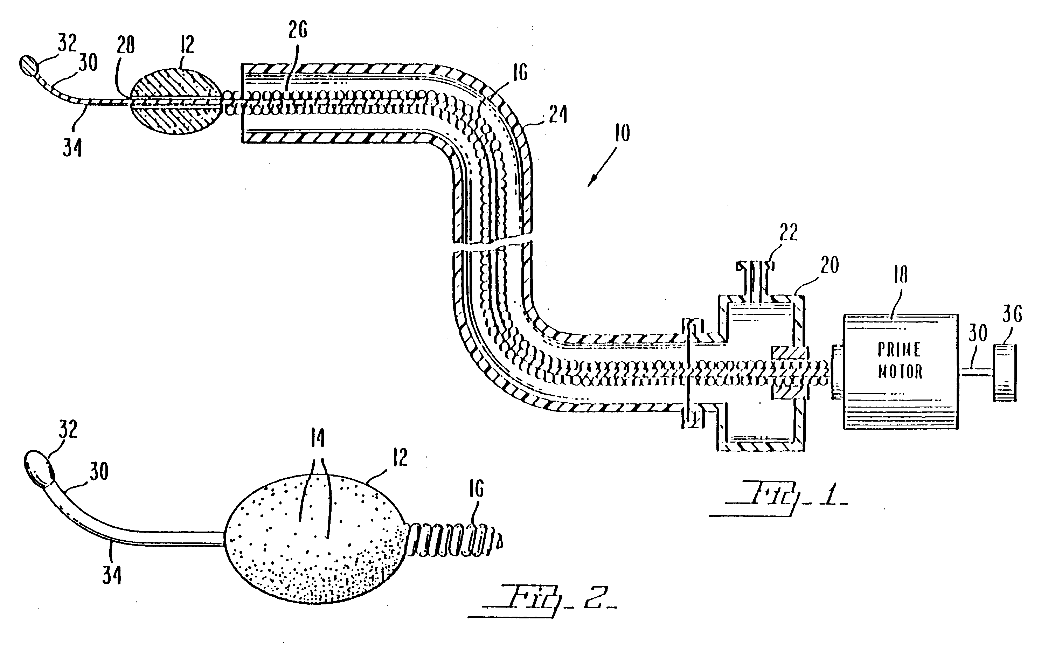

Referring generally to FIG. 1, the preferred embodiment 10 of the present invention is shown. The invention 10 comprises an abrasive burr 12 and an atraumatic tip 32 which is generally of the type described in US-A-4,646,736 which is steerable for accessing branch vessels. The burr 12 is covered with an abrasive cutting material, such as diamond grit 52, which is used in the preferred embodiment of the invention. The burr 12 is connected via a hollow, flexible drive shaft 16 to a variable speed prime mover 18. In the preferred embodiment of the invention, the drive shaft 16 is a 0,5 mm (.020") diameter trifilar helically wound drive shaft. The drive shaft 16 is sealably coupled to a variable speed rotational prime mover 18, which is capable of high speed rotation. The coupling is accomplished using a sealed chamber 20 having an injection port 22, so that injection of drugs or fluids into the lumen which is formed between the drive shaft 16 and a surrounding plastic sheath can be accommodated. The distal segment 25 of the flexible shaft 16 is preferrably passivated with a coating, of a low-friction material, such as du Pont's Teflon brand (registered trademark) tetrafluoroethylene homopolymer, which will inhibit the winding of intravascular fiber on the shaft 16 during rotation. The burr 12 includes a central bore 29 which is aligned with the opening which extends down the length of the hollow shaft 16. The burr 12 and the shaft 16 are routed into a vessel by using a central guide rail 30, which may be comprised of a 0,43 mm (.005") diameter steel wire. Adjacent the blunt tip 32 at the distal end of the guide rail 30, there is a preformable portion 34 of the guide rail 30 which the physician using the invention may bend to facilitate directing the invention into branch vessels. The guide rail 30 extends completely through the shaft 16 and through the prime mover 18 to a rotatable knob 36 which permits the guide rail 30 to be rotated in order to direct the tip 32 through a patient's vessel in order to perform a thrombectomy as described in US-A-4,679,557.

The drive shaft 16 and the central rail 30 may be individually moved with respect to each other and with respect to the plastic sheath 24 in order to engage a thrombus or an atheromatous occlusion. The rotational prime mover 18 for the high-speed helical drive shaft 16 is preferably operable in a range of from 20,000 rpm to greater than 155,000 rpm. The size of the burr tip 12 is typically in a range of from less than 1 mm diameter up to about 6 mm, depending upon the vessel size desired where the lesion is being recanalized.

Such a device provides for transluminal recanalization of intravascular lesions of soft or hard constitution consisting of thrombotic or atheromatous material.

Referring to FIG. 3, a preferred embodiment of the present invention utilizes a burr 50 having coated on the distal surface thereof, i.e., the portion most remote from the prime mover 18 (shown in FIG. 1), a variety of particles 52 ranging in size from about 30 µm (microns up) to about 150 µm (microns) in diameter. The smaller particles 56 are preferably located in the area 54 adjacent to the portion 58 of the burr 50 having the largest diameter, and the larger particles 60 are preferably located in the area 62 adjacent to the distal end 64 of the burr 50. Accordingly, when the burr 50 is inserted into a patient's vessel the larger particles 60 adjacent the distal end 64 serve to quickly abrade through any lodged material, thereby opening the vessel rapidly. The smaller particles 56 continue to abrade and polish the inner surface of the vessel as the burr 50 is advanced therethrough. The region 58 having the widest diameter is preferably substantially devoid of abrasive particles, so that it acts as a central bearing area. Accordingly, if the burr 50 is allowed to remain in a particular position within a patient's vessel, the absence of abrasive material in the central region 58 prevents that region from abrading through the wall of the patient's vessel.

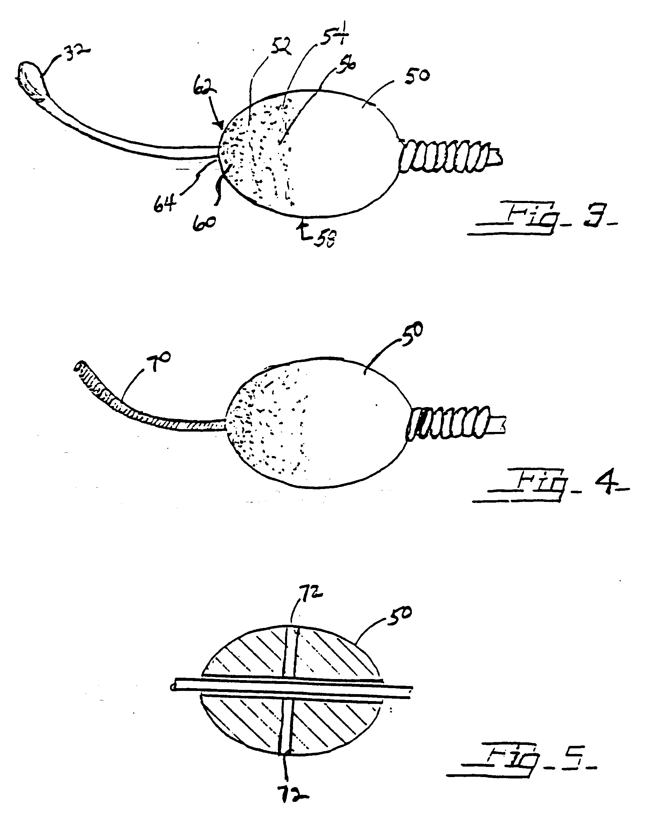

Referring to FIG. 4 the atraumatic tip 32 illustrated in FIGS. 1 and 3 can be replaced by preformable spring tip 70 of a type used in the catheter art. The preformable spring tip 70 can be bent by a physician, as desired, prior to insertion into a patient's vessel, in order that the unit may be guided through a patient's vessel to a particular location, generally under the assistance of fluoroscopy.

Referring generally to FIG. 5, radial openings 72 may be formed in the burr 50 to permit the outward flow of water (pumped in from the proximal end), whereby a friction reducing bearing will be accomplished.

## Claims

1. A transluminal microdissection device comprising
(a) a rotatable, flexible drive shaft (16) having a substantially ellipsoidal cutting burr (12;50) attached thereto, said burr having a diameter which is greater than that of said drive shaft (16);
(b) an abrasive surface on said burr (12;50), said abrasive surface being comprised of an abrasive material (14;52) attached only to the distal surface of said burr (12;50); and
(c) a rotatable prime mover (18) which is capable of rotating said drive shaft (16) at a rotation rate of from about 20,000 revolutions per minute to about 160,000 revolutions per minute, whereby particles small enough to pass through the capillaries of a person can be microdissected.

2. The transluminal microdissection device of Claim 1 wherein said abrasive material (60) adjacent the distal end of said burr is more coarse than said abrasive material (56) closer to the portion of said burr where said burr has its widest diameter.

3. The transluminal microdissection device of Claim 2 wherein the coarseness of said abrasive material is varied from finest to most coarse, with the most coarse abrasive material (60) adjacent the distal end of said burr and the finest abrasive material (56) closest to the portion of said burr having the widest diameter.

4. The transluminal microdissection device of Claim 3 wherein said abrasive material does not cover the surface of said burr (12;50) where said burr has its widest diameter.

5. The transluminal microdissection device of Claim 4 wherein said abrasive material does not cover the surface of said burr between the proximal end of said burr and the portion of said burr which has its widest diameter.

6. The transluminal microdissection device of any of Claims 1 to 5 wherein said drive shaft (16) and said burr (12;50) are hollow, whereby they may be guided along a guide wire (30).

7. The transluminal microdissection device of Claim 6 further comprising a tubular sheath (24) which surrounds said flexible drive shaft (16) and chamber means (20) for sealably attaching to said tubular sheath (24), said chamber means (20) including a seal through which said drive shaft (16) passes.

8. The transluminal microdissection device of Claim 7 further comprising a port (22) which extends into said chamber means (20) whereby there is a lumen formed between said drive shaft (16) and said sheath (24), and said lumen is accessible through said port (22).

9. The transluminal microdissection device of any of claims 6 to 8 wherein said guide wire (30) comprises a tip (32) at its distal end and/or a preformable portion (34) adjacent to said tip (32) at its distal end.

## Patentansprüche

1. Transluminales Mikroseziergerät mit
(a) einer drehbaren, biegsamen Antriebswelle (16) mit einem daran befestigten, im wesentlichen ellipsenförmigen Schneidfräser (12; 50), wobei der Fräser einen größeren Durchmesser als die Antriebswelle (16) aufweist;
(b) einer abrasiven Oberfläche auf dem Fräser (12; 50), wobei die abrasive Oberfläche mit einem abrasiven Material (14; 52) versehen ist, das nur auf der distalen Oberfläche des Fräsers (12; 50) befestigt ist; und
(c) einer drehbaren Hauptantriebseinrichtung (18), die die Antriebswelle (16) mit einer Drehgeschwindigkeit von ungefähr 20 000 U.p.M. bis ungefähr 160 000 U.p.M. drehen kann, wobei Teilchen, die klein genug sind, um durch die Kapillaren einer Person hindurchzugehen, mikroseziert werden können.

2. Transluminales Mikroseziergerät nach Anspruch 1, wobei das dem distalen Ende der Fräse benachbarte abrasive Material (60) rauher als das abrasive Material (56) ist, das näher zu dem Teil des Fräsers mit dem größten Durchmesser ist.

3. Transluminales Mikroseziergerät nach Anspruch 2, wobei die Rauhigkeit des abrasiven Materials von der geringsten bis zur größten Rauhigkeit variiert, das abrasive Material (60) mit der größten Rauhigkeit dem distalen Ende des Fräsers benachbart ist und das abrasive Material (56) mit der geringsten Rauhigkeit dem Abschnitt des Fräsers mit dem größten Durchmesser am nächsten ist.

4. Transluminales Mikroseziergerät nach Anspruch 3, wobei das abrasive Material nicht die Oberfläche des Fräsers (12; 50) an seinem größten Durchmesser bedeckt.

5. Transluminales Mikroseziergerät nach Anspruch 4, wobei das abrasive Material nicht die Oberfläche zwischen dem proximalen Ende des Fräsers und dem Teil des Fräsers mit dem weitesten Durchmesser bedeckt.

6. Transluminales Mikroseziergerät nach einem der Ansprüche 1 bis 5, wobei die Antriebswelle (16) und der Fräser (12; 50) hohl sind, wodurch sie längs eines Führungsdrahtes (30) geführt werden können.

7. Transluminales Mikroseziergerät nach Anspruch 6, ferner mit einer rohrförmigen Hülle (24), die die biegsame Antriebswelle (16) umgibt, und mit einer Kammereinrichtung (20), die abdichtbar an der rohrförmigen Hülle (24) befestigt ist, wobei die Kammereinrichtung (20) eine Dichtung aufweist, durch die die Antriebswelle (16) hindurchgeht.

8. Transluminales Mikroseziergerät nach Anspruch 7, ferner mit einer Öffnung (22), die sich in die Kammereinrichtung (20) erstreckt, wobei ein Freiraum zwischen der Antriebswelle (16) und der Hülle (24) ausgebildet ist und der Freiraum über die Öffnung (22) zugänglich ist.

9. Transluminales Mikroseziergerät nach einem der Ansprüche 6 bis 8, wobei der Führungsdraht (30) eine Spitze (32) an seinem distalen Ende und/oder einen vorformbaren Abschnitt (34) anschließend an die Spitze (32) an seinem distalen Ende aufweist.

## Revendications

1. Dispositif de microdissection transluminale, comprenant
(a) un arbre d'entraînement souple et tournant (16) auquel est fixée une fraise (12;50) de forme sensiblement ellipsoïdale, ladite fraise ayant un diamètre supérieur à celui dudit arbre d'entraînement (16);
(b) une surface abrasive sur ladite fraise (12;50), ladite surface abrasive étant constituée d'un matériau abrasif (14;52) fixé uniquement à la surface distale de ladite fraise (12;50); et
(c) un moteur primaire tournant (18) qui est capable de faire tourner ledit arbre d'entraînement (16) à une vitesse de rotation d'environ 20 000 tours par minute à environ 160 000 tours par minute de façon à permettre la microdissection des particules assez petites pour passer dans les capillaires d'une personne.

2. Dispositif de microdissection transluminale selon la revendication 1, dans lequel ledit matériau abrasif (60) proche de l'extrémité distale de ladite fraise est plus gros que ledit matériau abrasif (56) plus proche de la partie de ladite fraise où ladite fraise a son diamètre le plus grand.

3. Dispositif de microdissection transluminale selon la revendicaton 2, dans lequel la grosseur dudit matériau abrasif va du plus fin au plus gros, le matériau abrasif le plus gros (60) étant proche de l'extrémité distale de ladite fraise et le matériau abrasif le plus fin (56) étant le plus proche de la partie de ladite fraise de diamètre le plus grand.

4. Dispositif de microdissection transluminale selon la revendicaton 3, dans lequel ledit matériau abrasif ne recouvre pas la surface de ladite fraise (12;50) où ladite fraise a son diamètre le plus grand.

5. Dispositif de microdissection transluminale selon la revendicaton 4, dans lequel ledit matériau abrasif ne recouvre pas la surface de ladite fraise comprise entre l'extrémité proximale de ladite fraise et la partie de ladite fraise qui a le plus grand diamètre.

6. Dispositif de microdissection transluminale selon l'une quelconque des revendications 1 à 5, dans lequel ledit arbre d'entraînement (16) et ladite fraise (12;50) sont creux, ce qui permet leur guidage le long d'un fil de guidage (30).

7. Dispositif de microdissection transluminale selon la revendication 6, comprenant en outre une gaine tubulaire (24) qui entoure ledit arbre d'entraînement souple (16), et un compartiment (20) destiné à être fixé de façon étanche à ladite gaine tubulaire (24), ledit compartiment (20) comportant un joint d'étanchéité à travers lequel passe ledit arbre d'entraînement (16).

8. Dispositif de microdissection transluminale selon la revendication 7, comprenant en outre une lumière (22) qui débouche dans ledit compartiment (20), un espace libre étant formé entre ledit arbre d'entraînement (16) et ladite gaine (24), et ledit espace libre étant accessible par ladite lumière (22).

9. Dispositif de microdissection transluminale selon l'une quelconque des revendications 6 à 8, dans lequel ledit fil de guidage (30) comprend un embout (32) à son extrémité distale et/ou une partie préformable (34) à proximité dudit embout (32) à son extrémité distale.
